# EUROPÄISCHE PATENTSCHRIFT

(11) **EP 0 618 815 B1**
(45) Veröffentlichungstag und Bekanntmachung des Hinweises auf die Patenterteilung: **17.04.1996**
(21) Anmeldenummer: 93900055.0
(22) Anmeldetag: 15.12.1992
(51) Int. Cl.: A61K 39/395, C07K 16/40, C12P 21/08, C12N 5/00

(54) **MONOKLONALE ANTIKÖRPER GEGEN DIE TYP I-PHOSPHOLIPASE A 2? ALS ENTZÜNDUNGSHEMMENDES THERAPEUTIKUM**
MONOCLONAL ANTIBODIES AGAINST TYPE I PHOSPHOLIPASE A 2?, USED AS A THERAPEUTIC AGENT TO REDUCE INFLAMMATION
ANTICORPS MONOCLONAUX DIRIGES CONTRE LA PHOSPHOLIPASE A 2? DE TYPE I, UTILISES COMME AGENT THERAPEUTIQUE INHIBANT L'INFLAMMATION

(30) Priorität: 21.12.1991 DE 4142552
(43) Veröffentlichungstag der Anmeldung: 12.10.1994
(73) Patentinhaber: BOEHRINGER MANNHEIM GMBH, D-68298 Mannheim (DE)
(72) Erfinder: SCHEUER, Werner, D-8122 Penzberg (DE); HÜBNER-PARAJSZ, Christa, D-8132 Tutzing (DE); TIBES, Ulrich, D-6000 Frankfurt 70 (DE)
(74) Vertreter: Fouquet, Herbert, Dr.
(86) Internationale Anmeldenummer: EP9202905
(87) Internationale Veröffentlichungsnummer: WO9312816

(56) Entgegenhaltungen:
- EP-A- 0 287 397
- EP-A- 0 459 450
- BIOCHEMICAL AND BIOPHYSICAL RESEARCH COMMUNICATIONS, Band 167, Nr. 3, 1990, K. TAKAYAMA et al.; S. 1309-1315

## Beschreibung

Die Erfindung betrifft die Verwendung von monoklonalen Antikörpern gegen die Typ I-Phospholipase A₂ zur Herstellung eines entzündungshemmenden Therapeutikums, das sich insbesondere zur Anwendung bei akuter Pankreatitis eignet.

Für die akute Pankreatitis existiert weder eine kausale noch eine symptomatische Therapie (The Merck Manual of Diagnosis and Therapy 15 (1987), Seite 763-767). Pathogenetische Grundlage der akuten Pankreatitis ist eine Autolyse des Pankreas. Hierbei wird der Phospholipase A₂ eine entscheidende Funktion zugeschrieben. Sie wirkt lysierend auf die Zellmembran und setzt dabei aus Membranphospholipiden Arachidonsäure frei. Die Metabolite der Arachidonsäure (Prostaglandine und Leukotriene) sind wesentlicher Bestandteil der Entzündungsreaktion.

Die Phopholipase A₂ kommt in zwei Formen vor. Die als Typ I bezeichnete Phopholipase A₂ wird im wesentlichen im Pankreasgewebe gefunden und spielt bei der akuten Pankreatitis eine entscheidende Rolle. Dagegen ist die Typ II Phospholipase A₂ in vielen verschiedenen Geweben anzutreffen.

In der EP-A 0 248 597 werden niedermolekulare Hemmstoffe der Phospholipase A₂ beschrieben, die eine entzündungshemmende Wirkung bei der Maus aufweisen. Für eine etwa 80 %-ige Hemmung ist jedoch eine Applikation von 200 - 400 µg dieser Verbindungen notwendig. Bei derart hohen Dosierungen schränken Nebenwirkungen dieser niedermolekularen Inhibitoren deren therapeutische Anwendbarkeit deutlich ein. Diesen Nachteil weist auch der in der JP 088193 beschriebene Inhibitor der Phospholipase A₂ auf, der mit einer täglichen Dosierung von 100 bis 1000 mg bei erwachsenen Patienten zur Therapie einer Pankreatitis vorgeschlagen wird. Die in der EP-A 0 405 864 beschriebenen Phospholipase A₂-Inhibitoren aus dem Mikroorganismus Circinotrichum falcatisporum weisen für die Hemmung von gereingter Rattenphospholipase A₂ IC₅₀-Werte von 17,5 bis über 300 µg/ml auf und müßten somit bei einer therapeutischen Anwendung ebenfalls sehr hoch dosiert werden.

Als weiterer Inhibitor der Phospholipase A₂ weist auch das 37 kD große Lipocortin eine entzündungshemmende Wirkung auf (B. Wallner et.al., Nature 320 (1986), 77-81). Wegen seiner geringen Stabilität eignet sich Lipocortin jedoch nicht für eine therapeutische Verwendung. In der EP-A 0 327 334 werden daher 15-26 Aminosäuren große Peptide beschrieben, die eine hemmende Wirkung auf die Phospholipase A₂ aufweisen. Lediglich bei einem dieser Peptide liegt jedoch der IC₅₀-Wert unter 10 µg/ml. Es wird jedoch nicht gezeigt, daß dieses Peptid aus 16 ungeschützten Aminosäuren eine höhere Stabilität als Lipocortin aufweist.

Von K. Takayama et.al. (Biochem. Biophys. Res. Comm. 167 (1990), 1309-1315) werden monoklonale Antikörper gegen die menschliche Phospholipase A₂ aus der Synovialflüssigkeit beschrieben, die jedoch nicht an die Phospholipase A₂ aus dem Pankreas binden.

In EP-A 0 287 397 und J. Clin. Biochem. Nutr. 11 (1991), 79 - 89 werden monoklonale Antikörper gegen die Phospholipase A₂ aus dem Pankreas beschrieben. Einige dieser Antikörper hemmen die enzymatische Aktivität der Phospholipase A₂ mit einer IC₅₀ von 0,2 ng/ml. Es wird jedoch lediglich eine diagnostische Verwendung dieser Antikörper beschrieben.

Aufgabe der vorliegenden Erfindung war es dagegen, einen Hemmstoff für die Phospholipase A₂ zur Verfügung zu stellen, dessen inhibitorische Wirkung die Verwendung dieses Hemmstoffs als Therapeutikum gegen die akute Pankreatitis ermöglicht.

Diese Aufgabe wird gelöst durch die Verwendung von monoklonalen Antikörpern gegen die Typ I-Phospholipase A₂, die bei einer Konzentration von weniger als 100 ng/ml eine mindestens 50 %-ige Hemmung der Aktivität der Phospholipase A₂ bewirken, oder funktioneller Fragmente dieser Antikörper, zur Herstellung eines Therapeutikums, das bei Entzündungserscheinungen, speziell bei akuter Pankreatitis eingesetzt werden kann.

Es hat sich überraschenderweise gezeigt, daß mit einem monoklonalen Antikörper gegen die Typ I Phospholipase A₂ oder einem funktionellen Derivat eines solchen Antikörpers ein besonders effizientes Therapeutikum gegen Entzündungsreaktionen, insbesondere gegen akute Pankreatitis, Psoriasis, Peritonitis oder Sepsis erhalten werden kann. Neben den vollständigen Antikörpern sind hierfür auch funktionelle Antikörperfragmente wie monoklonale Fab- oder F(ab')-Fragmente, sowie divalente F(ab')₂-Fragmente geeignet.

Ein weiterer Gegenstand der Erfindung ist daher eine pharmazeutische Formulierung, bestehend aus einem monoklonalen Antikörper gegen die Typ I-Phospholipase A₂ oder einem funktionellen Derivat dieses Antikörpers, gegebenenfalls zusammen mit den üblichen pharmazeutischen Träger-, Füll-, Hilfs- und Zusatzstoffen, sowie ein Verfahren zur Herstellung eines solchen Arzneimittels, das insbesondere zur Therapie der akuten Pankreatitis eingesetzt werden kann.

Für die erfindungsgemäße Verwendung haben sich die aus den Hybridomlinien DSM ACC2026 und DSM ACC2025 erhältlichen monoklonalen Antikörper als besonders geeignet erwiesen.

Ein weiterer bevorzugter Gegenstand der Erfindung sind daher monoklonale Antikörper gegen die Typ I-Phospholipase A₂, die in äquivalenter Weise an die Typ I-Phospholipase A₂ bindefähig sind, wie die aus den Zellinien DSM ACC2026 und/oder DSM ACC2025 erhältlichen monoklonalen Antikörper gegen die Typ I-Phospholipase A₂.

Unter dem Begriff "in äquivalenter Weise bindefähige Antikörper" werden Antikörper verstanden, bei denen eine Epitopüberlappung mit dem definierten bekannten Antikörper nachweisbar ist. Diese Epitopüberlappung kann mit Hilfe eines kompetitiven Testsystems leicht nachgewiesen werden. Dazu wird zum Beispiel mit Hilfe eines Enzymimmunoassays überprüft, in wie weit ein Antikörper mit dem bekannten Antikörper um die Bindung an ein definiertes Antigen bzw. ein spezielles Epitop konkurriert. Dazu inkubiert man das entsprechende Antigen mit dem bekannten monoklonalen Antikörper in markierter Form und einem Überschuß des in Betracht gezogenen Antikörpers. Durch Immobilisierung der gebildeten Komplexe, Trennung der festen von der flüssigen Phase und Nachweis der gebundenen Markierung in einer der beiden Phasen, kann dann leicht festgestellt werden, in wie weit der in Betracht gezogene Antikörper den definierten Antikörper aus der Bindung verdrängen kann. Ist eine Verdrängung von mindestens 50 % bei 10⁵-fachen Überschuß gegeben, so liegt eine Epitopüberlappung vor.

Besonders bevorzugt sind die monoklonalen Antikörper gegen die Typ I-Phospholipase A₂, die aus den Zellinien DSM ACC2026 und/oder DSM ACC2025 erhältlich sind.

Ein weiterer Gegenstand der Erfindung sind die Zellinien DSM ACC2026 und/oder DSM ACC2025.

Die erfindungsgemäßen monoklonalen Antikörper sind erhältlich durch Immunisierung mit gereinigter Phospholipase A₂, Immortalisieren von Milzzellen der immunisierten Tiere und Klonierung derjenigen immortalisierten Zellen, deren Kulturüberstand einen Antikörper enthält, der eine Hemmung der Aktivität der Phospholipase A₂ mit einer IC₅₀ von weniger als 100ng/ml bewirkt. Die von diesen Klonen produzierten monoklonalen Antikörpern werden dann nach bekannten Verfahren isoliert.

Die Immunisierung wird in den hierfür üblicherweise verwendeten Tieren wie z.B. Mäusen oder Ratten durchgeführt. Vorzugsweise werden Mäuse verwendet.

Die Immortalisierung der Milzzellen der immunisierten Tiere erfolgt vorzugsweise durch Fusionierung mit der Myelomzellinie P3X63-Ag 8.653 (ATCG CRL 1580) gemäß der Methode nach J. of Imm. Meth. 39 (1980), 285-308. Daneben können aber auch andere, dem Fachmann geläufige Verfahren zur Immortalisierung der Milzzellen verwendet werden.

Zur Klonierung werden die Zellen zum Beispiel mittels eines floureszenzsaktivierten Zellsorters vereinzelt. Zum Nachweis von immortalisierten Zellen, die den gewünschten Antikörper gegen Phospholipase A₂ produzieren, wird eine Probe des Kulturüberstandes in einem ELISA-Test auf Reaktivität mit der Phospholipase A₂ getestet. Um solche Antikörper zu erhalten, welche die enzymatische Aktivität der Phospholipase A₂ hemmen, wird der Kulturüberstand derjenigen Klone, die an die Phospholipase A₂ bindende Antikörper produzieren, zusätzlich auf die Hemmung der Phospholipase A₂-Aktivität in einem enzymatischen Test untersucht.

Diejenigen Klone, deren Kulturüberstand die gewünschte Hemmung der Phospholipase A₂-Aktivität ergibt, werden expandiert und die von diesen Klonen produzierten Antikörper nach bekannten Verfahren isoliert.

Die einen erfindungsgemäßen monoklonalen Antikörper gegen die Phospholipase A2 produzierenden Hybridomzellinien DSM ACC2026 und DSM ACC2025 wurden am 10.12.1991 bei der Deutschen Sammlung von Zellkulturen und Mikroorganismen GmbH, Mascheroder Weg 1 b, D-3300 Braunschweig, hinterlegt.

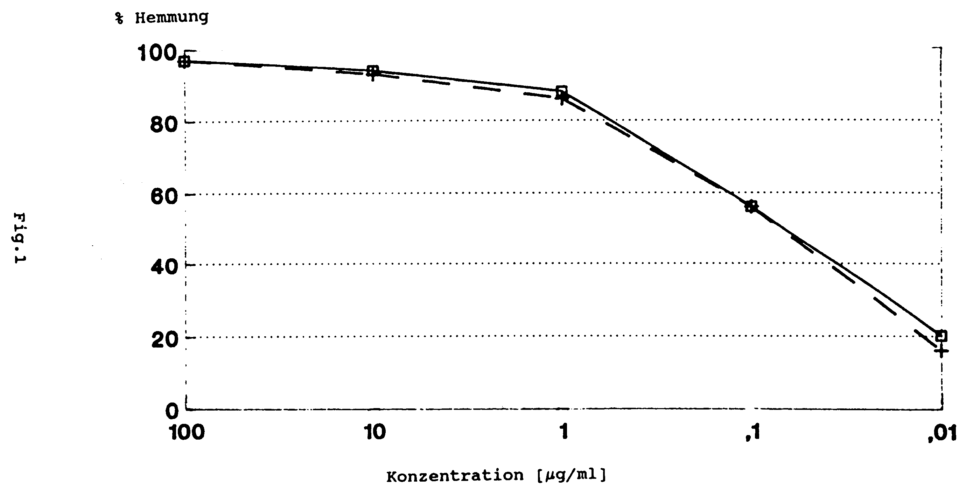

Die Erfindung wird durch die folgenden Beispiele in Verbindung mit der Figur 1 erläutert.

Figur 1 zeigt die Hemmung der Phospholipase A₂-Aktivität durch die monoklonalen Antikörper DSM ACC2026 (+) und DSM ACC2025
(□).

### Beispiel 1:

### Herstellung von monoklonalen Antikörpern gegen die Typ I-Phospholipase A₂

12 Wochen alte Balb/C-Mäuse werden intraperitoneal mit 50 µg gereinigter Phospholipase A₂ (EG 3.1.1.4, V. Kozumplik et al., Biochim. Biophys. Acta 1002 (1989), 395 - 397) in komplettem Freund's Adjuvans immunisiert. Zwei nachfolgende Injektionen von jeweils 50 µg werden im Abstand von jeweils einem Monat mit inkomplettem Freund's Adjuvans gegeben. 3 Tage vor der Zellfusion erfolgt eine intravenöse Injektion von weiteren 50 µg in physiologischer Kochsalzlösung.

Die Fusion der Milzzellen der immunisierten Mäuse mit der Myelomzellinie P3X63-Ag8.653 (ATCC CRL 1580) erfolgt gemäß einer Modifikation der ursprünglich von Köhler und Milstein beschriebenen Methode (J. of Imm. Meth. 39 (1980), 285-308). Die fusionierten Zellen werden in RPMI 1640 Medium (das 10 % FKS,2 mmol/l L-Glutamin und 1 mmol/l Natriumpyruvat, sowie 0,1 mmol/l Hypoxantin und 10 µmol/l Azaserin enthält) kultiviert.

Positive Primärkulturen (Bestimmung gemäß Beispiel 2 und 3) werden 2 Wochen nach Fusion mit Hilfe eines floureszenzaktivierten Zellsorters kloniert. Die Zellen werden dabei einzeln in 96-er Mikrotiterplatten abgelegt.

### Beispiel 2:

### Bestimmung der Spezifität der produzierten Antikörper

Um die Spezifität der Antikörper im Kulturüberstand der Hybridomzellen zu erfassen, wird ein Enzyme Linked Immunosorbent Assay (ELISA) angewendet.

Dazu werden 96-er Mikrotiterplatten mit 100 µl Phospholipase A₂ Antigen (5 µg/ml in Carbonatpuffer, Boehringer Mannheim GmbH, Katalog-Nr. 726 559) beschichtet, mit 100 µl Kulturüberstand (1:25 mit PBS (nach Dulbecco und Vogt, J. Exp. Meth. 99 (1954), 167-182) verdünnt) 2 Stunden bei Raumtemperatur inkubiert und mit 3 x 350 µl PBS/0,05 % Tween® 20 gewaschen. Nach Zugabe der den Antikörper enthaltenden Probelösung wird eine Stunde bei Raumtemperatur inkubiert und erneut gewaschen. Danach wird mit POD-markiertem Schaf-anti-Maus-Immunglobulin G (10 mU, Boehringer Mannheim GmbH, Katalog-Nr. 13 17 377) 1 Stunde bei Raumtemperatur inkubiert, mit 3 x 350 µl PBS/0,05 % Tween 20 gewaschen und die Nachweisreaktion durch Zugabe von 100 µl ABTS® (1 mg/ml, Boehringer Mannheim GmbH, Katalog-Nr. 756 407) in 40 mmol/l Citratpuffer pH 4,4 der 3,25 mmol/l Natriumperborat enthält (Boehringer Mannheim GmbH, Katalog-Nr. 1204 530) ausgelöst. Nach 30 Minuten Inkubation bei Raumtemperatur wird die Extinktion in einem ELISA-Reader bei 405 nm bestimmt.

### Beispiel 3:

### Test auf Inhibierung der enzymatischen Aktivität

Humane duodenale Phospholipase A₂ (s. Beispiel 1) wird mit Trispuffer aus der Testkombination "Freie Fettsäuren" (Boehringer Mannheim GmbH, Katalog-Nr. 1056 239) verdünnt.

20 µl dieser Enzymlösung wird 15 min. bei 25°C mit 10 µl der zu untersuchenden Antikörperlosung in verschiedenen Konzentrationen (s. Tab. 1) inkubiert. Anschließend wird 20 µl Substrat (Lecithinemulsion aus der Testkombination "Freie Fettsäuren", Boehringer Mannheim GmbH, Katalog-Nr. 1056 239) zugegeben und 60 Minuten bei 37°C inkubiert. Die hierbei durch die Phospholipase A₂-Aktivität freigesetzten Fettsäuren werden mittels der Testkombination "Freie Fettsäuren" (Boehringer Mannheim GmbH, Katalog-Nr. 1383 175) durch Messung der Extinktion gemäß Angaben des Herstellers quantifiziert. Die Ergebnisse sind in %-Hemmung im Vergleich zur Enzymaktivität ohne Antikörper in der folgenden Tabelle 1, sowie in Figur 1, dargestellt (jeweils Mittelwerte aus 3 Bestimmungen).

**Tabelle 1**

| Probe | Konz (µg/ml) | %-Hemmung |
|---|---|---|
| DSM ACC2025 | 100 | 97 |
| | 10 | 93 |
| | 1 | 86 |
| | 0,1 | 56 |
| | 0,01 | 16 |
| DSM ACC2026 | 100 | 97 |
| | 10 | 94 |
| | 1 | 88 |
| | 0,1 | 56 |
| | 0,01 | 20 |

### Beispiel 4:

### Bestimmung der Epitopüberlappung von Antikörpern gegen Phospholipase A₂

Zum Nachweis der Epitopüberlappung eines Antikörpers mit dem monoklonalen Antikörper DSM ACC 2025 oder DSM ACC2026 wird ein kompetitiver Enzymimmunoassay durchgeführt.

Dazu wird Phospholipase A₂ zunächst mit D-Biotinyl-ε-amidocapronsäure-N-hydroxysuccinimidester (Boehringer Mannheim GmbH, Katalog-Nr. 1008 960) gemäß Angaben des Herstellers biotinyliert. Von diesem biotinylierten Antigen werden 300 ng in einem Volumen von 100 µl PBS durch einstündige Inkubation bei Raumtemperatur an eine mit Streptavidin beschichtete Mikrotiterplatte (Herstellung nach EP-A 0 344 578) gebunden. Nach viermaligem Waschen mit PBS/0,05 % Tween® 20 wird 90 Minuten bei Raumtemperatur simultan inkubiert mit dem monoklonalen Antikörper DSM ACC2025 bzw. DSM ACC2026, der mit Peroxidase markiert wurde (Endkonzentration 250 mU/ml) und dem zu beurteilenden Antikörper. Nach erneutem viermaligem Waschen mit PBS/0,05 % Tween® 20 wird mit der Enzymsubstratlösung ABTS® in Natriumperborat enthaltendem Puffer (s. Beispiel 2) 30 Minuten bei Raumtemperatur inkubiert und anschließend die Extinktion bei 405 nm als Maß für die Menge des gebundenen POD-markierten monoklonalen Antikörpers DSM ACC2025 bzw. DSM ACC2026 gemessen. Dieser Wert wird verglichen mit der Extinktion, die erhalten wird, bei Inkubation mit dem monoklonalen Antikörper DSM ACC2025 bzw. DSM ACC2025 allein. Wenn bis zum einem 10⁵-fachen Überschuß an zu beurteilendem Antikörper gegenüber dem monoklonalen Antikörper DSM ACC2025 bzw. DSM ACC2026 als Enzymkonjugat (250 mU/ml) mindestens 50 % Kompetition zu erkennen sind, liegt eine Epitopüberlappung vor.

### Beispiel 5:

### Inhibierung der PLA₂-TypI-induzierten PGE₂- und LTC₄-Ausschüttung durch humane Leukozyten in Gegenwart eines monoklonalen Antikörpers gegen die Phospholipase A₂

Humane Leukozyten werden durch Dichtezentrifugation (Lymphozytentrennmedium der Boehringer Mannheim GmbH, Kat.-Nr. 295949) aus peripherem Blut isoliert und auf einen Zelltiter von 0,75 x 10⁶ Zellen/ml in 125 mmol/l Trispuffer aus der Testkombination "Freie Fettsäuren" (Boehringer Mannheim GmbH, Katalog-Nr. 1056 239) eingestellt. Verschiedene Konzentrationen von gereinigter humaner Phospholipase A₂ (siehe Beispiel 1 und Tabelle 2) werden mit einer Lösung des monoklonalen Antikörpers DSM ACC2026 (Konzentration siehe Tabelle 2) und den humanen Leukozyten für 4 h bei 37°C inkubiert. Anschließend werden die Zellen für 10 Min. bei 800 g abzentrifugiert und in den Überständen die PGE₂- und LTC₄-Konzentration über einen Radioimmunoassay (Biermann, Bad Nauheim, Deutschland, für PGE₂ und NEN DuPont, Dreieich, Deutschland, für LTC₄) bestimmt. Die Ergebnisse von jeweils zwei unabhängigen Versuchen (humane Leukozyten von zwei verschiedenen Spendern) sind in der nachfolgenden Tabelle 2 angegeben (jeweils Mittelwerte von drei Bestimmungen). In den positiven Kontrollansätzen (Inkubation mit Phospholipase A₂ Typ I, aber ohne monoklonalen Antikörper gegen diese Phospholipase A₂) ist deutlich die PLA₂-induzierte Stimulierung der Ausschüttung der Entzündungsmediatoren PGE₂ und LTC₄ durch humane periphere Lymphozyten zu erkennen. Diese in vivo zu einer Entzündungsreaktion führende Ausschüttung von PGE₂ und LTC₄ kann durch Zugabe eines monoklonalen Antikörpers gegen die TypI-Phospholipase A₂ deutlich gehemmt werden.

**Tabelle 2**

| PLA₂-Typ I | MAK〈PLA₂〉 DSM ACC2026 | PGE₂ [ng/ml] | | LTC₄ [ng/ml] | |
|---|---|---|---|---|---|
| | | Exp. 1 | Exp. 2 | Exp. 1 | Exp. 2 |
| - | - | 0,82 | 1,99 | 0,17 | 0,19 |
| 1 mg/ml | - | 5,42 | 9,13 | 0,87 | 0,91 |
| -"- | 0.1 mg/ml | 2,18 | 4,13 | 0,24 | 0,25 |
| -"- | 0.01 mg/ml | 3,23 | 5,20 | 0,31 | 0,28 |
| -"- | 0.001 mg/ml | 4,72 | 8,89 | 0,49 | 0,77 |
| 0,1 mg/ml | - | 2,16 | 4,44 | 0,23 | 0,32 |
| -"- | 0.1 mg/ml | 0,97 | 2,53 | 0,17 | 0,20 |
| -"- | 0.01 mg/ml | 1,29 | 2,74 | 0,16 | 0,19 |
| -"- | 0.001 mg/ml | 1,63 | 3,13 | 0,21 | 0,17 |
| 0,01 mg/ml | - | 1,05 | 2,44 | 0,17 | 0,15 |
| -"- | 0.1 mg/ml | 0,74 | 2,17 | 0,13 | 0,13 |
| -"- | 0.01 mg/ml | 0,82 | 1,99 | 0,13 | 0,12 |
| -"- | 0.001 mg/ml | 0,97 | 1,73 | 0,14 | 0,14 |

## Patentansprüche

1. Verwendung eines monoklonalen Antikörpers gegen die Typ I Phospholipase A₂, der bei einer Konzentration von weniger als 100 ng/ml eine mindestens 50 %-ige Hemmung der Aktivität der Phospholipase A₂ bewirkt, oder eines funktionellen Fragments dieses Antikörpers zur Herstellung eines Therapeutikums, das bei Entzündungserscheinungen, speziell bei akuter Pankreatitis eingesetzt werden kann.

2. Pharmazeutische Formulierung, bestehend aus einem monoklonalen Antikörper gegen die Typ I Phospholipase A₂, der bei einer Konzentration von weniger als 100 ng/ml eine mindestens 50 %-ige Hemmung der Aktivität der Phospholipase A₂ bewirkt, oder eines funktionellen Fragments dieses Antikörpers zusammen mit den üblichen pharmazeutischen Träger-, Füll-, Hilfs- und Zusatzstoffen.

3. Monoklonaler Antikörper gegen die Typ-I-Phospholipase A₂, der bei einer Konzentration von weniger als 100 ng/ml eine mindestens 50 %-ige Hemmung der Aktivität der Phospholipase A₂ bewirkt und der in äquivalenter Weise an die Typ-I-Phospholipase A₂ bindefähig ist, wie ein aus den Zellinien DSM ACC2026 und/oder DSM ACC2025 erhältlicher monoklonaler Antikörper.

4. Monoklonaler Antikörper gegen die Typ-I-Phospholipase A₂, der bei einer Konzentration von weniger als 100 ng/ml eine mindestens 50 %-ige Hemmung der Aktivität der Phospholipase A₂ bewirkt und erhältlich ist aus der Zellinie DSM ACC2026 oder DSM ACC2025.

5. Zellinie DSM ACC2026.

6. Zellinie DSM ACC2025.

## Claims

1. Use of monoclonal antibodies against type I phospholipase A₂ which, at a concentration of less than 100 ng/ml, brings about an at least 50% inhibition of the activity of phospholipase A₂ or of a functional fragment of this antibody for the preparation of a therapeutic which can be used in the case of inflammatory phenomena, especially in the case of acute pancreatitis.

2. Pharmaceutical formulation consisting of a monoclonal antibody against type I phospholipase A₂ which, at a concentration of less than 100 ng/ml, brings about an at least 50% inhibition of the activity of phospholipase A₂ or of a functional fragment of this antibody, together with the usual pharmaceutical carrier, filling, adjuvant and additive materials.

3. Monoclonal antibody against type I phospholipase A₂ which, at a concentration of less than 100 ng/ml, brings about an at least 50% inhibition of the activity of phospholipase A₂ and which, in equivalent manner, is bindable to type I phospholipase A₂ like a monoclonal antibody obtainable from the cell lines DSM ACC2026 and/or DSM ACC2025.

4. Monoclonal antibody against type I phospholipase A₂ which, at a concentration of less than 100 ng/ml, brings about an at least 50% inhibition of the activity of phospholipase A₂ and is obtainable from the cell lines DSM ACC2026 or DSM ACC2025.

5. Cell line DSM ACC2026.

6. Cell line DSM ACC2025.

## Revendications

1. Utilisation d'un anticorps monoclonal dirigé contre la phospholipase A₂ de type I, qui, à une concentration inférieure à 100 ng/ml, inhibe l'activité de la phospholipase A₂ dans une proportion d'au moins 50%, ou d'un fragment fonctionnel de cet anticorps pour la préparation d'un agent thérapeutique qui peut être utilisé dans des affections de type inflammatoire, en particulier dans le cas d'une pancréatite aiguë.

2. Formulation pharmaceutique, constituée d'un anticorps monoclonal dirigé contre la phospholipase A₂ de type I, qui, à une concentration inférieure à 100 ng/ml, inhibe l'activité de la phospholipase A₂ dans une proportion d'au moins 50 %, ou d'un fragment fonctionnel de cet anticorps, conjointement avec des véhicules, charges, adjuvants et agent auxiliaires pharmaceutiques usuels.

3. Anticorps monoclonal dirigé contre la phospholipase A₂ de type I, qui, à une concentration inférieure à 100 ng/ml, inhibe l'activité de la phospholipase A₂ dans une proportion d'au moins 50 % et qui est capable de se lier à la phospholipase A₂ de type I de manière équivalente à celle d'un anticorps monoclonal que l'on peut obtenir à partir de la lignée cellulaire DSM ACC2026 et/ou DSM ACC2025.

4. Anticorps monoclonal dirigé contre la phospholipase A₂ de type I, qui, à une concentration inférieure à 100 ng/ml, inhibe l'activité de la phospholipase A₂ dans une proportion d'au moins 50 % et que l'on peut obtenir à partir de la lignée cellulaire DSM ACC2026 et/ou DSM ACC2025.

5. Lignée cellulaire DSM ACC2026.

6. Lignée cellulaire DSM ACC2025.
